(19) Europäisches Patentamt
European Patent Office
Office européen des brevets

(11) Veröffentlichungsnummer: **0 368 187 B1**

(12) **EUROPÄISCHE PATENTSCHRIFT**

(45) Veröffentlichungstag der Patentschrift: **01.09.93**

(51) Int. Cl.[5]: **C07K 15/00**, A61K 37/02

(21) Anmeldenummer: **89120462.0**

(22) Anmeldetag: **06.11.89**

Die Akte enthält technische Angaben, die nach dem Eingang der Anmeldung eingereicht wurden und die nicht in dieser Patentschrift enthalten sind.

# (54) Neue Insulinderivate, ihre Verwendung und eine sie enthaltende pharmazeutische Zubereitung.

(30) Priorität: **08.11.88 DE 3837825**

(43) Veröffentlichungstag der Anmeldung:
**16.05.90 Patentblatt 90/20**

(45) Bekanntmachung des Hinweises auf die Patenterteilung:
**01.09.93 Patentblatt 93/35**

(84) Benannte Vertragsstaaten:
**AT BE CH DE ES FR GB GR IT LI LU NL SE**

(56) Entgegenhaltungen:
**EP-A- 0 046 979**
**EP-A- 0 133 285**
**EP-A- 0 254 516**

(73) Patentinhaber: **HOECHST AKTIENGESELLSCHAFT**

**D-65926 Frankfurt(DE)**

(72) Erfinder: **Dörschug, Michael, Dr.**
**Sonnenleite 20**
**D-4630 Bochum(DE)**

**EP 0 368 187 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Insulin und Insulinderivate werden bekanntlich in erheblichen Mengen zur Behandlung der Krankheit Diabetes mellitus benötigt und z.T. auch großtechnisch produziert. Trotz der beträchtlichen Zahl der bereits existierenden Insulin-Zubereitungen und -Abwandlungen mit unterschiedlichen Wirkungsprofilen besteht wegen der Verschiedenheit der Organismen mit deren inter- und intra-individuellen Schwankungen immer noch ein Bedarf an weiteren Insulinprodukten mit wieder anderen Eigenschaften und Wirkungscharakteristiken.

Insulinderivate mit einer verzögerten Wirkung sind z.B. beschrieben in EP-B-132 769 und EP-B-132 770. Es handelt sich um speziell in der Position B31 der Insulin-B-Kette basisch modifizierte Derivate der folgenden Formel I:

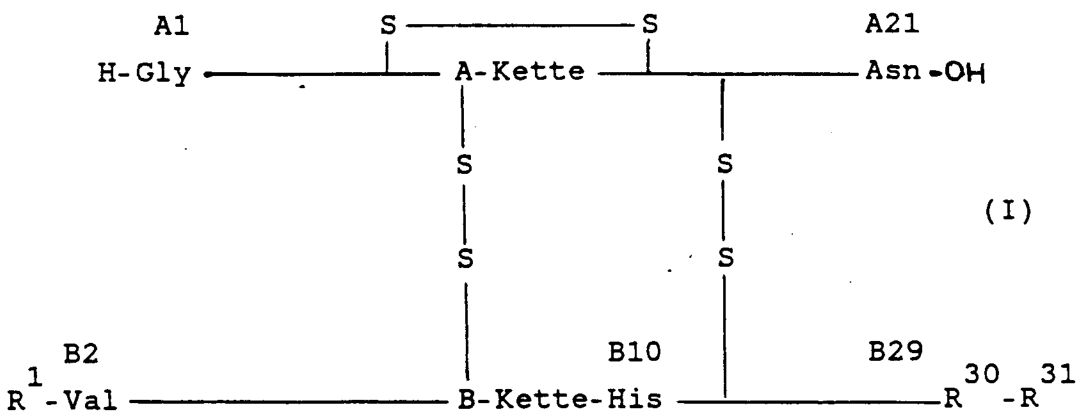

(I)

in welcher $R^1$ H oder H-Phe bedeutet,
$R^{30}$ für den Rest einer neutralen, genetisch kodierbaren L-Aminosäure steht und
$R^{31}$ für eine physiologisch unbedenkliche organische Gruppe basischen Charakters mit bis zu 50 C-Atomen steht, an deren Aufbau 0 bis 3 $\alpha$-Aminosäuren beteiligt sind und deren gegebenenfalls vorhandene endständige Carboxylfunktion frei, als Esterfunktion, als Amidfunktion, als Lacton oder zu $CH_2OH$ reduziert vorliegen kann.

Für diese Insulinderivate ist ein isoelektrischer Punkt zwischen 5,8 und 8,5 (gemessen in der isoelektrischen Fokussierung) charakteristisch. Der - gegenüber dem isoelektrischen Punkt des unmodifizierten nativen Insulins oder Proinsulins (bei pH = 5,4) in den Neutralbereich hinein verschobene - isoelektrische Punkt ist durch die zusätzliche(n), an der Oberfläche des Moleküls befindliche(n) positive(n) Ladung(en) infolge der basischen Modifikation bedingt. Damit sind diese basisch modifizierten Insulinderivate im Neutralbereich weniger löslich als etwa natives Insulin oder Proinsulin, welche im Neutralbereich normalerweise gelöst vorliegen.

Die Verzögerungs- oder Depot-wirkung der basisch modifizierten Insulinderivate der Formel I geht auf deren Schwerlöslichkeit am isoelektrischen Punkt zurück. Die Wiederauflösung der Insulinderivate unter physiologischen Bedingungen soll nach den beiden vorerwähnten Druckschriften durch Abspaltung der zusätzlichen basischen Gruppen erreicht werden, was je nach Derivat durch tryptische oder Trypsin-ähnlich und/oder Carboxypeptidase B oder der Carboxypeptidase B-ähnliche und/oder Esterase-Aktivität zustande kommt. Die jeweils abgespaltenen Gruppen sind entweder rein physiologische Metaboliten oder aber leicht metabolisierbare, physiologisch unbedenkliche Substanzen.

Das vorerwähnte Depotprinzip infolge basischer Modifikation des Insulins wurde noch weiter genutzt durch die Bereitstellung und entsprechende Verwendung anderer - hauptsächlich innerhalb der A- und B-Ketten - basisch modifizierter Insulinderivate; vgl. z.B. EP-A-0194 864 und EP-A-0254 516.

In den Insulinderivaten gemäß EP-A-0194 864 ist in B27-Position eine basische Aminosäure eingebaut und/oder eine neutrale Aminosäure sitzt in den Positionen A4, A17, B13 und/oder B21; außerdem ist die C-terminale Carboxylgruppe der B-Kette durch einen Amid- oder Esterrest blockiert.

Die Insulinderivate gemäß EP-A-0254 516 sind denen gemäß der vorher genannten EP-A sehr ähnlich; zwecks Stabilitätserhöhung bei den schwach sauren pH-Werten der entsprechenden pharmazeutischen Zubereitungen kann jedoch hier die Aminosäure Asn in Position A21 noch durch andere, in saurem Medium stabilere Aminosäuren wie z.B. Asp, ersetzt sein bzw. werden. Asn (= Asparagin) unterscheidet sich von Asp (=Asparaginsäure) bekanntlich durch die Blockierung einer der beiden Carboxylgruppen durch die

Amidgruppe:

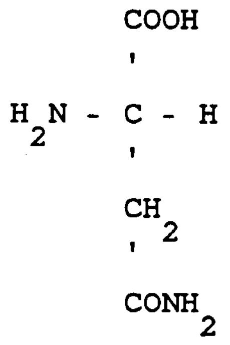

**Asparagin**

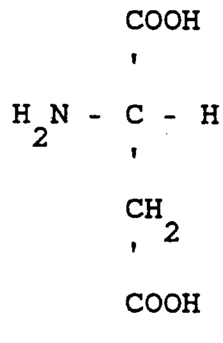

**Asparaginsäure**

Durch eine wieder andere Modifikation des Insulinmoleküls in der A- und B-Kette, insbesondere durch Austausch des für die Komplexbildung mit Zink - und damit für eine gewisse Verzögerungswirkung verantwortliche - Aminosäure His in B10-Position durch anderen entsprechende Aminosäuren sollen schnell wirkende Insulinderivate resultieren; vgl. EP-A-0214 826.

Sämtliche Insulinderivate gemäß den 3 zuletzt genannten Druckschriften sind hauptsächlich innerhalb der A- und B-Ketten modifiziert; ihre Herstellung erfolgt auf gentechnologischem Wege.

In dem Bestreben, die Stabilität der am C-terminalen Ende der B-Kette basisch modifizierten Insulinderivate gemäß den eingangs erwähnten EP-Patentschriften EP-B-0132 769 und EP-B-0132 770 in saurem Medium zu erhöhen und gegebenenfalls auch deren Wirkungsprofil noch zu verändern, wurde nun gefunden, daß dieses Ziel in vorteilhafter Weise erreicht wird
durch Ersatz von $Asn^{21}$ durch andere genetisch kodierbare Aminosäuren, welche keine Amidgruppe enthalten, und gegebenenfalls durch Substitution von $His^{B10}$ durch anderen genetisch kodierbare Aminosäuren.

Erfindungsgegenstand sind daher Insulinderivate der Formel II

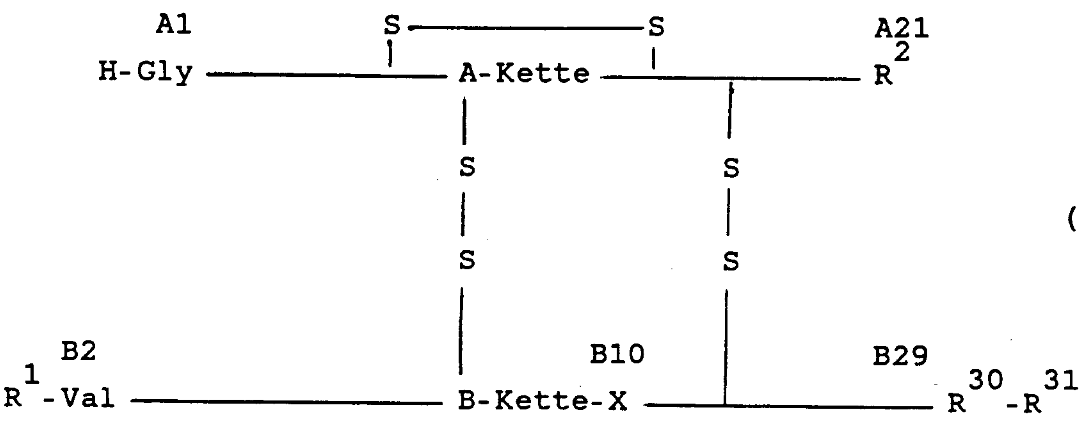

(II)

in welcher

- $R^1$ H oder H-Phe bedeutet,
- $R^2$ eine genetisch kodierbare, keine Amidgruppe enthaltende L-Aminosäure bedeutet,
- $R^{30}$ für den Rest einer neutralen, genetisch kodierbaren L-Aminosäure steht,
- $R^{31}$ für eine physiologisch unbedenkliche organische Gruppe basischen Charakters mit bis zu 50 C-Atomen steht, an deren Aufbau 0 bis 3 $\alpha$-Aminosäuren beteiligt sind und deren gegebenenfalls vorhandene endständige Carboxylfunktion frei, als Esterfunktion, als Amidfunktion, als Lacton oder zu $CH_2OH$ reduziert vorliegen kann, und
- X für eine genetisch kodierbare L-Aminosäure steht

mit einem isoelektrischen Punkt zwischen 5 und 8,5 und deren physiologisch verträglichen Salze.

Die neuen Insulinderivate und deren physiologisch verträgliche Salze sind bei den schwach sauren pH-Werten entsprechender pharmazeutischer Zubereitungen auch über längere Zeiträume stabil und besitzen - insbesondere wenn auch $His^{B10}$ noch durch andere Aminosäuren ausgetauscht ist - ein gegenüber den bekannten - unveränderten - basisch modifizierten Insulinderivaten der am Anfang angegebenen Formel I verändertes (kürzeres) Wirkungsprofil.

In Formel II ist $R^1$ vorzugsweise H-Phe.

Genetisch kodierbare, keine Amidgruppe enthaltende L-Aminosäuren - für $R^2$ - sind Gly, Ala, Ser, Thr, Val, Leu, Ile, Asp, Glu, Cys, Met, Arg, Lys, His, Tyr, Phe, Trp, Pro; bevorzugt sind Gly, Ala, Ser, Thr, Asp und Glu, insbesondere Asp.

Neutrale, genetisch kodierbare L-Aminosäuren - für $R^{30}$ - sind Gly, Ala, Ser, Thr, Val, Leu, Ile, Asn, Gln, Cys, Met, Tyr, Phe und Pro; bevorzugt sind Ala, Thr und Ser.

$R^{31}$ ist eine physiologisch unbedenklich organische Gruppe basischen Charakters mit bis zu 50 C-Atomen, an deren Aufbau 0 - 3 $\alpha$-Aminosäuren beteiligt sind. Wenn am Aufbau von $R^{31}$ keine $\alpha$-Aminosäuren beteiligt sind, kommen für diesen Rest z.B. folgende basischen Gruppen in Frage: Amino-($C_2$-$C_6$-)-alkoxy, $C_1$-$C_4$)-Alkylamino-($C_2$-$C_6$)-alkoxy, Di-($C_1$-$C_4$)-alkylamino-($C_2$-$C_6$)-alkoxy, Tri-($C_1$-$C_4$)-ammonio-($C_2$-$C_6$)-alkoxy, Amino-($C_2$-$C_6$)-alkylamino, [($C_1$-$C_4$)-Alkylamino]-($C_2$-$C_6$)-alkylamino, Di-($C_1$-$C_4$)-alkylamino-($C_2$-$C_6$)-alkylamino oder [Tri-($C_1$-$C_4$)-alkylamino]-($C_2$-$C_6$)-alkylamino, insbesondere $-O-[CH_2]_p-NR_2$, $O-[CH_2]_p-N^{\oplus}R_3$, $-NH-[CH_2]_p-NR_2$ oder $-NH-[CH_2]_p-N^{\oplus}R_3$, worin p = 2 bis 6 ist und R gleich oder verschieden ist und für Wasserstoff oder ($C_1$-$C_4$)-Alkyl steht.

Wenn am Aufbau von $R^{31}$ bis zu 3 $\alpha$-Aminosäuren beteiligt sind, sind dies in erster Linie neutrale oder basische natürlich vorkommende L-Aminosäuren und/oder die diesen entsprechenden D-Aminosäuren. Neutral natürlich vorkommende Aminosäuren sind insbesondere Gly, Ala, Ser, Thr, Val, Leu, Ile, Asn, Gln, Cys, Met, Tyr, Phe, Pro und Hyp. Basische natürlich vorkommende Aminosäuren sind insbesondere Arg, Lys, Hyl, Orn, Cit und His. Falls nur neutrale $\alpha$-Aminosäuren beteiligt sind, kann deren endständige Carboxylfunktion - damit $R^{31}$ basischen Charakter hat - nicht frei sein; die Carboxylfunktion muß vielmehr in diesem Fall mit einer basischen Gruppe verestert oder amidiert sein, wobei als solche basischen Gruppen etwa die vorher - für den Fall, daß am Aufbau von $R^{31}$ keine $\alpha$-Aminosäuren beteiligt sind - erwähnten basischen Gruppen in Frage kommen. Natürlich können diese basischen Ester- oder Amidgruppen auch die Carboxylfunktion von basischen $\alpha$-Aminosäuren blockieren. Für die Blockierung der Carboxylfunktion der basischen $\alpha$-Aminosäuren können - falls die Blockierung gewünscht ist - auch neutrale Ester- oder Amidgruppen wie z.B. ($C_1$-$C_6$)-Alkoxy, ($C_3$-$C_6$)-Cycloalkyloxy, $NH_2$, ($C_1$-$C_6$)-alkylamino oder Di-($C_1$-$C_6$)-alkylamino in Frage kommen.

Als Lacton kann die endständige Carboxylfunktion natürlich nur vorliegen, wenn die endständige Aminosäure eine Hydroxyaminosäure ist.

Außerdem kann die endständige Carboxylfunktion auch zu $CH_2OH$ reduziert sein.

Bevorzugt besteht $R^{31}$ aus 1, 2 oder 3 der vorerwähnten basischen natürlich vorkommenden Aminosäuren; besonders bevorzugt ist $R^{31}$ = Arg-OH oder Arg-Arg-OH.

Als genetisch kodierbare L-Aminosäuren - für X - kommen die gleichen Aminosäuren wie für $R^2$ in Frage, wobei hier jedoch auch noch die genetisch kodierbaren L-Aminosäuren, welche eine Amidgruppen enthalten - d.s. Asn und Gln - möglich sind; letztere - Asn und Gln - sind hier sogar bevorzugt. Wenn Asn oder Gln sich in Position B10 befinden, ist die Amidgruppe jedenfalls in schwach saurem Medium stabil (im Gegensatz zu Asn oder Gln in Position A21.)

Die Sequenzen (A1 - A20) und (B1 - B9, B11 - B29) sind vorzugsweise die Sequenzen des Human-, Schweine- oder Rinder-Insulins, insbesondere die Sequenzen des Human-Insulins.

Beispielhafte Insulin-Derivate der Formel II sind:

$Asp^{A21}$-Humaninsulin-$Arg^{B31}$-OH
$Glu^{A21}$- "
$Gly^{A21}$- "
$Ser^{A21}$- "
$Thr^{A21}$- "
$Ala^{A21}$- "

$Asp^{A21}$-Humaninsulin-$Arg^{B31}$-$Arg^{B32}$-OH
$Glu^{A21}$- "
$Gly^{A21}$- "
$Ser^{A21}$- "
$Thr^{A21}$- "
$Ala^{A21}$- "

$Asp^{A21}$-$Asn^{B10}$-Humaninsulin-$Arg^{B31}$-OH
$Glu^{A21}$- "
$Gly^{A21}$- "
$Ser^{A21}$- "
$Thr^{A21}$- "
$Ala^{A21}$- "

$Asp^{A21}$-$Asn^{B10}$-Humaninsulin-$Arg^{B31}$-$Arg^{B32}$-OH
$Glu^{A21}$- "
$Gly^{A21}$- "
$Ser^{A21}$- "
$Thr^{A21}$- "
$Ala^{A21}$- "

Die Herstellung der Insulin-Derivate der Formel II erfolgt hauptsächlich gentechnologisch mittels site-directed mutagenesis nach Standardmethoden.

Dazu wird eine für das gewünschte Insulin-Derivat der Formel II codierende Genstruktur konstruiert und in einer Wirtszelle - vorzugsweise in einem Bakterium wie E. coli oder einer Hefe, insbesondere Saccharomyces cerevisiae - zur Expression genracht und - falls die Genstruktur für ein Fusionsprotein codiert - aus dem Fusionsprotein das Insulin-Derivat der Formel II freigesetzt; analoge Methoden sind z.B. beschrieben in EP-A-0 211 299, EP-A-0 227 938, EP-A-0 229 998, EP-A-0 286 956 und der DE-Patentanmeldung P 38 21 159.9 vom 23.6.1988 (HOE 88/F 158).

Die Abspaltung des Fusionsproteinanteils erfolgt nach Zellaufschluß entweder chemisch mittels Halogencyan - vgl. EP-A-0 180 920 - oder enzymatisch mittels Lysostaphin - vgl. DE-A-37 39 347.

Der Insulinvorläufer wird dann der oxidativen Sulfitolyse nach der z.B. von R.C. Marshall und A.S. Inglis in "Practical Protein Chemistry - A Handbook" (Herausgeber A. Darbre) 1986, S. 49 - 53 beschriebenen Methode unterworfen
und anschließend in Gegenwart eines Thiols unter Ausbildung der korrekten Disulfidbrücken renaturiert, z.B. nach der von G.H. Dixon und A.G. Wardlow in Nature (1960), S. 721 - 724 beschriebenen Methode.

Das C-Peptid wird mittels tryptischer Spaltung entfernt - z.B. gemäß der Methode von Kemmler et al., J.B.C. (1971), S. 6786 - 6791
und das Insulinderivat der Formel II mittels bekannter Techniken wie Chromatographie - vgl. z.B. EP-A-0 305 760 - und Kristallisation gereinigt.

Die Herstellung der Insulin-Derivate der Formel II mit $R^2$ = Asp und X = His erfolgt zweckmäßig durch Hydrolyse der bekannten basisch modifizierten Insulin-Derivate der Formel I in wäßrig-saurem Medium (weil hier nur die Amidgruppe des Asparagins in Position A21 hydrolysiert werden muß), vorzugsweise bei pH-Werten zwischen etwa 2 und etwa 4, insbesondere von etwa 2,5, und bei Temperaturen von etwa 0 bis etwa 40 °C, vorzugsweise bei Raumtemperatur.

Die erfindungsgemäßen Insulin-Derivate der Formel II und/oder deren physiologisch verträgliche Salze (wie z.B. die Alkali- oder Ammoniumsalze) werden hauptsächlich als Wirkstoffe für eine pharmazeutische Zubereitung zur Behandlung des Diabetes mellitus verwendet.

Die pharmazeutische Zubereitung ist vorzugsweise eine Lösung oder Suspension zu Injektionszwecken; sie ist gekennzeichnet durch einen Gehalt an mindestens einem Insulin-Derivat der Formel II und/oder mindestens einem von deren physiologisch verträglichen Salzen in gelöster, amorpher und/oder kristalliner - vorzugsweise in gelöster - Form.

Die Zubereitung weist vorzugsweise einen pH-Wert zwischen etwa 2,5 und 8,5, insbesondere zwischen etwa 4,0 und 8,5, auf,
enthält ein geeignetes Isotoniemittel,
ein geeignetes Konservierungsmittel
und gegebenenfalls einen geeigneten Puffer,
sowie vorzugsweise auch eine bestimmte Zinkionen-Konzentration,
alles natürlich in steriler wäßriger Lösung. Die Gesamtheit der Zubereitungsbestandteile außer dem Wirkstoff bildet den Zubereitungs-Träger.

Geeignete Isotoniemittel sind z.B. Glycerin, Glukose, Mannit, NaCl, Calcium- oder Magnesium-Verbindungen wie $CaCl_2$, $MgCl_2$ etc.

Durch die Wahl des Isotoniemittels und/oder Konservierungsstoffes beeinflußt man die Löslichkeit des Insulin-Derivats bzw. dessen physiologisch verträglichen Salzes bei den schwach sauren pH-Werten.

Geeignete Konservierungsmittel sind z.B. Phenol, m-Cresol, Benzylalkohol und/oder p-Hydroxybenzoesäureester.

Als Puffersubstanzen, insbesondere zur Einstellung eines pH-Wertes zwischen etwa 4,0 und 8,5, können z.B. Natriumacetat, Natriumcitrat, Natriumphosphat etc. verwendet werden. Ansonsten sind zur Einstellung des pH-Wertes auch physiologisch unbedenkliche verdünnte Säuren (typischerweise HCl) bzw. Laugen (typischerweise NaOH) geeignet.

Wenn die Zubereitung einen Zinkgehalt besitzt, ist ein solcher von 1 µg bis 2 mg, insbesondere von 5 ug bis 200 µg Zink/ml bevorzugt.

Zwecks Variation des Wirkungsprofils der erfindungsgemäßen Zubereitung kann auch unmodifiziertes Insulin, vorzugsweise Rinder-, Schweine- oder Human-Insulin, insbesondere Human-Insulin, zugemischt werden.

Bevorzugte Wirkstoffkonzentrationen sind solche entsprechend etwa 1 - 1500, weiter bevorzugt etwa 5 - 1000 und insbesondere etwa 40 - 400 internationale Einheiten/ml.

Die Erfindung wird nun durch die folgenden Beispiele näher erläutert.

A) Gentechnologische Herstellung

### Beispiel 1

Konstruktion eines Plasmides zur Herstellung von Gly (A21)-Humaninsulin Arg (B31-OH)

In der DE-Patentanmeldung P 38 21 159.9 (HOE 88/F 158) wurde das Plasmid pSW3 beschrieben.
Die Plasmid-DNA wird mit den Restriktionsenzymen Pvu2 und Sal1 umgesetzt und anschließend mit alkalischer Rinderphosphatase behandelt. Die beiden entstehenden Fragmente werden gelelektrophoretisch getrennt und das große Fragment isoliert. Dieses Fragment wird in einer T4-DNA-Ligasereaktion mit folgender synthetischen DNA-Sequenz verknüpft:

```
5'- CTG GAA AAC TAC TGT GGT TGA TAG
    GAC CTT TTG ATG ACA CCA ACT ATC AGCT - 5'
```

Kompetente E.coli W3110 Zellen werden mit dem Ligationsansatz transformiert. Das Transformationsgemisch wird auf NA-Platten, die 20 µg Ap ( = Ampicillin)/ml enthalten, ausplattiert und über Nacht bei 37 °C inkubiert. Von einzelnen Kolonien wird eine Übernachtkultur gewonnen und aus dieser Plasmid-DNA gewonnen. Diese DNA wird mittels Restriktionsanalyse und DNA-Sequenzanalyse charakterisiert. Richtige Plasmide, die die veränderte A-Kette codieren, erhalten die Bezeichnung pIK100. Die Expression erfolgt analog Bsp. 3 der vorerwähnten DE-Patentanmeldung P 38 21 159.9. Die Herstellung des modifizierten Mono-Arg-Insulins erfolgt ebenfalls analog der in dieser DE-Patentanmeldung beschriebenen Herstellung des unmodifizierten Mono-Arg-Insulins.

**Beispiel 2**

Konstruktion eines Plasmides zur Herstellung von Ser(A21)-Humaninsulin (Arg B31-OH)

Die Konstruktion entspricht dem im obigen Beispiel beschriebenen Weg. Die synthetische DNA-Sequenz ist aber wie folgt abgewandelt:

```
5'- CTG GAA AAC TAC TGT TCA TGA TAG
    GAC CTT TTG ATG ACA AGT ACT ATC AGCT - 5'
```

Man erhält das Plasmid pIK110, das durch eine zusätzlich BspH1-Erkennungssequenz charakterisiert ist.

**Beispiel 3**

Konstruktion eines Plasmids zur Herstellung von Gly(A21)-Asn(B10)-Humaninsulin-Arg(B31-OH)

DNA des Plasmides pIK100 wird mit den Restriktionsenzymen HpaI und Dra3 gespalten und mit alkalischer Rinderphosphatase behandelt. Die beiden entstehenden Fragmente werden gelelektrophoretisch getrennt und das größere der beiden Fragmente isoliert. Das Fragment wird mit der synthetischen DNA-Sequenz

```
5' - AAC CAA CAC TTG TGT GGT TCT AAC TTG
     TTG GTT GTG AAC ACA CCA AGA TTG         - 5'
```

ligiert und kompetente E. coli W3110-Zellen mit dem Ligationsgemisch transformiert. Die weitere Charakterisierung des entstandenen Plasmides pIK101 erfolgt wie in Beispiel 1 beschrieben.

**Beispiel 4**

Konstruktion eines Plasmides zur Herstellung von Ser(A21)-Asn(B10)-Humaninsulin

Die Konstruktion entspricht der in Beispiel 3 beschriebenen Klonierung, jedoch wird von DNA des Plasmides pIK110 ausgegangen.
Das neu konstruierte Plasmid erhält die Bezeichnung pIK111.

**Beispiel 5**

Konstruktion eines Expressionsplasmides für Affenproinsulin

Affenproinsulin unterscheidet sich von humanem Proinsulin lediglich durch den Austausch einer einzigen Aminosäure im C-Peptid (B37-Pro anstelle von Leu in dieser Position des humanen Proinsulins).

Das Plasmid pSW3 wird mit Hpa1 und Sal1 geöffnet und die Restplasmid-DNA isoliert. Aus dem in der EP-A-0 229 998 beschriebenen Plasmid pK50 wird das Dra3-Sal1-Affenproinsulinfragment isoliert. Die beiden Fragmente werden mit dem synthetischen DNA-Fragment

```
5' - AAC CAG CAC CTG TGC GGT TCT CAC CTA
     TTG GTC GTG GAC ACG CCA AGA GTG      - 5'
```

in einer T4-DNA-Ligasereaktion verknüpft. Man erhält das Plasmid pSW2, dessen DNA im folgenden als Ausgangsmaterial für die Konstruktionen der die Di-Arg-Humaninsulinderivate kodierenden Expressionsplasmide dient.

**Beispiel 6**

Konstruktion eines Plasmides zur Herstellung von Gly(A21)-Humaninsulin Arg(B31)-Arg(B32)-OH

DNA des Plasmides pSW2 wird entsprechend Beispiel 1 mit Pvu2 und Sal1 gespalten und mit der synthetischen DNA aus Beispiel 1 ligiert; es entsteht das Plasmid pSW21.

**Beispiel 7**

Konstruktion eines Plasmides zur Herstellung von Ser(A21)-Humaninsulin-Arg(B31)-Arg(B32)-OH

Ausgehend von pSW2-DNA wird analog zu Beispiel 2 das Plasmid pSW22 konstruiert.

**Beispiel 8**

Konstruktion eines Plasmides zur Herstellung von Gly(A21)-Asn(B10)-Humaninsulin-Arg(B31)-Arg(B32)-OH

Ausgehend von pSW21-DNA wird analog zu Beispiel 3 das Plasmid pSW23 konstruiert.
Als synthetische DNA-Sequenz wird dabei folgende Sequenz verwendet:

```
5' - AAC CAA CAC TTG TGT GGT TCT AAC CTA
     TTG GTT GTG AAC ACA CAA AGA TTG      - 5'
```

**Beispiel 9**

Konstruktion eines Plasmides zur Herstellung von Ser(A21)-Asn(B10)-Humaninsulin-B31(Arg)-B32(Arg)-OH

Ausgehend von pSW22-DNA wird analog Beispiel 4 unter Verwendung der in Beispiel 8 beschriebenen synthetischen DNA-Sequenz das Plasmid pSW24 konstruiert.

B) **Herstellung von Asp$^{A21}$-Humaninsulin-Arg$^{B31}$-Arg$^{B32}$-OH**aus Humaninsulin-Arg$^{B31}$-Arg$^{B32}$-OH mittels Hydrolyse

1 g Humaninsulin-Arg$^{B31}$-Arg$^{B32}$-OH wird in 100 ml $H_2O$ aufgeschlämmt. Durch Zugabe von HCl wird der pH-Wert auf 2,5 eingestellt und die Lösung bei 37 °C belassen. Nach einer Woche ist ca. die Hälfte des Materials zu Asp$^{A21}$-Humaninsulin-Arg$^{B31}$-Arg$^{B32}$-OH umgesetzt. Das Produkt wird in an sich bekannter

Weise über einen Anionenaustauscher vom Ausgangsmaterial abgetrennt, aus dem Eluat gefällt und in einem Puffer, der pro Liter 10,5 g Citronensäure, 1 g Phenol und 5 ml einer 1 %igen Zinkchloridlösung enthält mit einer Proteinkonzentration von 5 g/l bei pH 6,0 kristallisiert. Die Ausbeute beträgt 390 mg $Asp^{A21}$-$Humaninsulin^{B31}$-$Arg^{B32}$.

C) **Herstellung einer Injektionslösung**

Das Insulin-Derivat gemäß B wird mit einer Konzentration von 1,4 mg/ml in einer sterilen Trägerlösung folgender Zusammensetzung (pro ml) gelöst:
18 mg Glycerin, 10 mg Benzylalkohol, 80 µg $Zn^{2+}$ , pH 4,0.

D) **Wirkprofil einer $Asp^{A21}$-Humaninsulin-$Arg^{B31}$-$Arg^{B32}$-OH-Zubereitung** am Hund im Vergleich zu Humaninsulin-$Arg^{B31}$-$Arg^{B32}$-OH und Basal-H-Insulin $Hoechst^{(R)}$ = eine NPH-(Neutral-Protamin nach Hagedorn) Zubereitung mit ca. 10 µg $Zn^{2+}$.

| Präparat | | Blutzucker in Prozent des Anfangswertes in Stunden (h) 1 h | 2 h | 3 h | 5 h | 7 h |
|---|---|---|---|---|---|---|
| erfindungsgemäß | $Asp^{A21}$-Humaninsulin $Arg^{B31}$-$Arg^{B32}$-OH | 99 | 62 | 51 | 75 | 98 |
| Vergleich | Humaninsulin $Arg^{B31}$-$Arg^{B32}$-OH | 77 | 52 | 64 | 85 | 98 |
| | Basal-H-Insulin $Hoechst^{(R)}$ | 71 | 49 | 59 | 83 | 100 |

Dieses Beispiel zeigt, daß $Asp^{A21-}$Humaninsulin-$Arg^{B31}$-$Arg^{B32}$-OH das gleiche vorteilhafte Basalprofil zeigt wie Humaninsulin-$Arg^{B31}$-$Arg^{B32}$-OH. Zusätzlich hat $Asp^{A21}$-Humaninsulin-$Arg^{B31}$-$Arg^{B32}$-OH die vorteilhafte Eigenschaft, daß die Verbindung unter den gewählten Bedingungen langzeitstabil ist.

**Patentansprüche**

**Patentansprüche für folgende Vertragsstaaten : AT, BE, CH, DE, FR, GB, IT, LI, LU, NL, SE**

**1.** Insulin-Derivate der Formel II,

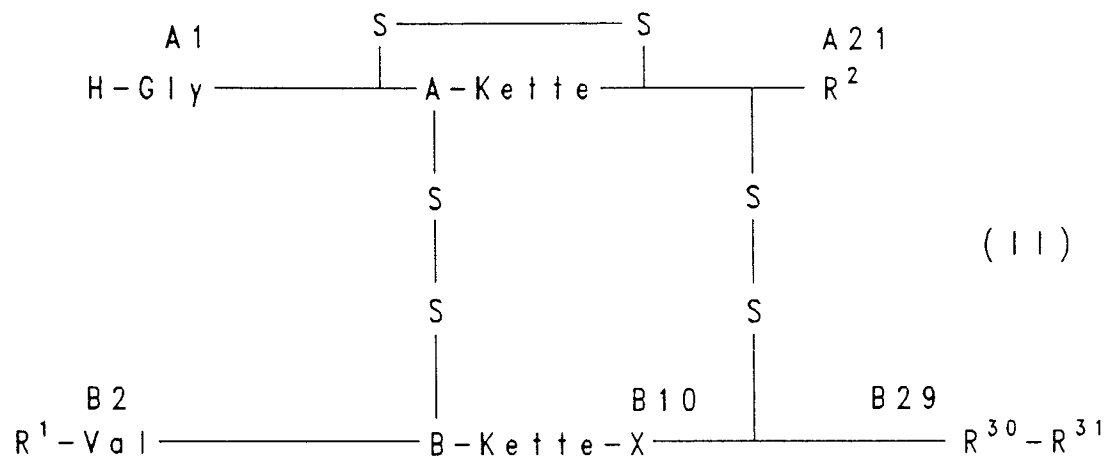

(II)

in welcher

- $R^1$ H oder H-Phe bedeutet,
- $R^2$ eine genetisch kodierbare, keine Amidgruppe enthaltende L-Aminosäure bedeutet,
- $R^{30}$ für den Rest einer neutralen, genetisch kodierbaren L-Aminosäure steht,
- $R^{31}$ für einen physiologisch unbedenklichen organischen Rest aus der Gruppe: Amino-($C_2$-$C_6$)-alkoxy, ($C_1$-$C_4$)-Alkylamino-($C_2$-$C_6$)-alkoxy, Di-($C_1$-$C_4$)-alkylamino-($C_2$-$C_6$)-alkoxy, Tri-($C_1$-$C_4$)-amonio-($C_2$-$C_6$)-alkoxy, Amino-($C_2$-$C_6$)-alkylamino [($C_1$-$C_4$)-Alkyl-amino]-($C_2$-$C_6$)-alkylamino, Di-($C_1$-$C_4$)-alkylamino-($C_2$-$C_6$)-alkylamino oder [Tri-($C_1$-$C_4$)-alkylamino]-($C_2$-$C_6$)-alkylamino, insbesondere $-O-[CH_2]_p-NR_2$, $-O-[CH_2]_p-N^{\oplus}R_3$, $-NH[CH_2]_p-NR_2$ oder $-NH-[CH_2]_p-N^{\oplus}R_3$, worin p = 2 bis 6 ist und R gleich oder verschieden ist und für Wasserstoff oder ($C_1$-$C_4$)-Alkyl steht oder
  für 1 bis 3 $\alpha$-Aminosäuren steht, deren gegebenenfalls vorhandene endständige Carboxyfunktion frei, als Esterfunktion, als Amidfunktion, als Lacton oder zu $CH_2OH$ reduziert vorliegen kann, und
- X für eine genetisch kodierbare L-Aminosäure steht,

gekennzeichnet durch einen isoelektrischen Punkt zwischen 5 und 8,5, und deren physiologisch verträglichen Salze.

**2.** Insulin-Derivate und deren physiologisch verträgliche Salze nach Anspruch 1, dadurch gekennzeichnet, daß in Formel II $R^1$ für H-Phe steht.

**3.** Insulin-Derivate und deren physiologisch verträgliche Salze nach Anspruch 1 oder 2, dadurch gekennzeichnet, daß in Formel II $R^2$ für Gly, Ala, Ser, Thr, Asp oder Glu, insbesondere nur für Asp, steht.

**4.** Insulin-Derivate und deren physiologisch verträgliche Salze nach einem oder mehreren der Ansprüche 1 bis 3, dadurch gekennzeichnet, daß in Formel II $R^{30}$ für Ala, Thr oder Ser steht.

**5.** Insulin-Derivate und deren physiologisch verträgliche Salze nach einem oder mehreren der Ansprüche 1 bis 4, dadurch gekennzeichnet, daß in Formel II $R^{31}$ für Arg-OH oder Arg-Arg-OH steht.

**6.** Insulin-Derivate und deren physiologisch verträgliche Salze nach einem oder mehreren der Ansprüche 1 bis 5, dadurch gekennzeichnet, daß in Formel II X Asn oder Gln bedeutet.

**7.** Insulin-Derivate und deren physiologisch verträgliche Salze nach einem oder mehreren der Ansprüche 1 bis 6, dadurch gekennzeichnet, daß in Formel II die Sequenzen (A1 bis A20) und (B1 bis B9, B11 bis B29) die Sequenzen des Human-, Schweine- oder Rinderinsulins, insbesondere die Sequenzen des Humaninsulins sind.

8. Verwendung der Insulin-Derivate und deren physiologisch verträgliche Salze gemäß einem oder mehreren der Ansprüche 1 bis 7 als Wirkstoffe für pharmazeutische Zubereitungen zur Behandlung des Diabetes mellitus.

9. Pharmazeutische Zubereitung, gekennzeichnet durch eine wirksame Menge an mindestens einem Insulin-Derivat der Formel II und/oder mindestens einem von deren physiologisch verträglichen Salzen gemäß einem oder mehreren der Ansprüche 1 bis 7 in gelöster, amorpher und/oder kristalliner - vorzugsweise in gelöster - Form.

10. Pharmazeutische Zubereitung nach Anspruch 9, gekennzeichnet durch einen zusätzlichen Gehalt von 1 µg bis 2 mg, vorzugsweise 5 µg bis 200 µg Zink/ml.

11. Pharmazeutische Zubereitung nach Anspruch 9 oder 10, gekennzeichnet durch einen zusätzlichen Gehalt an unmodifiziertem Insulin, vorzugsweise unmodifiziertem Humaninsulin.

**Patentansprüche für folgende Vertragsstaaten : ES, GR**

1. Verfahren zur Herstellung von Insulin-Derivate der Formel II,

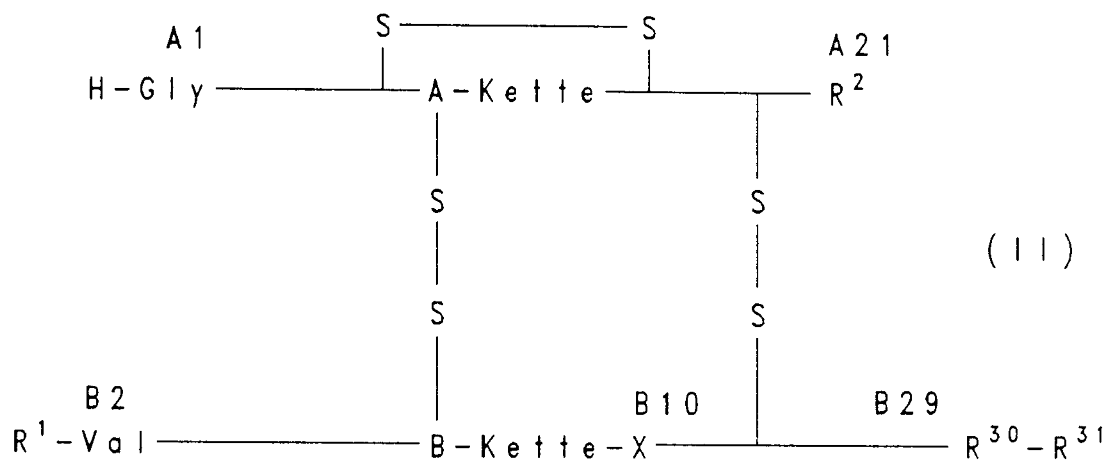

(II)

in welcher

- $R^1$ H oder H-Phe bedeutet,
- $R^2$ eine genetisch kodierbare, keine Amidgruppe enthaltende L-Aminosäure bedeutet,
- $R^{30}$ für den Rest einer neutralen, genetisch kodierbaren L-Aminosäure steht,
- $R^{31}$ für einen physiologisch unbedenklichen organischen Rest aus der Gruppe: Amino-($C_2$-$C_6$)-alkoxy, ($C_1$-$C_4$)-Alkylamino-($C_2$-$C_6$)-alkoxy, Di-($C_1$-$C_4$)-alkylamino-($C_2$-$C_6$)-alkoxy, Tri-($C_1$-$C_4$)-amonio-($C_2$-$C_6$)-alkoxy, Amino-($C_2$-$C_6$)-alkylamino [($C_1$-$C_4$)-Alkyl-amino]-($C_2$-$C_6$)-alkylamino, Di-($C_1$-$C_4$)-alkylamino-($C_2$-$C_6$)-alkylamino oder [Tri-($C_1$-$C_4$)-alkylamino]-($C_2$-$C_6$)-alkylamino, insbesondere -O-[$CH_2$]-$NR_2$, O-[$CH_2$]$_p$-$N^{\oplus}R_3$, -NH[$CH_2$]$_p$-$NR_2$ oder -NH-[$CH_2$]$_p$-$N^{\oplus}R_3$, worin p = 2 bis 6 ist und R gleich oder verschieden ist und für Wasserstoff oder ($C_1$-$C_4$)-Alkyl steht oder
  für 1 bis 3 α-Aminosäuren steht, deren gegebenenfalls vorhandene endständige Carboxyfunktion frei, als Esterfunktion, als Amidfunktion, als Lacton oder zu $CH_2OH$ reduziert vorliegen kann, und
- X für eine genetisch kodierbare L-Aminosäure steht,

mit einem isoelektrischen Punkt zwischen 5 und 8,5, und der physiologisch verträglichen Salze dieser Insulin-Derivate, dadurch gekennzeichnet, daß man für diese Insulin-Derivate codierende Genstrukturen in einer Wirtszelle, vorzugsweise in einem Bakterium oder in einer Hefe, zur Expression bringt und - falls die Genstrukturen für ein Fusionsprotein codieren - aus dem erhalten Fusionsprotein das jeweilige Insulin-Derivat der Formel II freisetzt und gegebenenfalls in ein physiologisch verträgliches Salz überführt.

2. Verfahren nach Anspruch 1, dadurch gekennzeichnet, daß man Insulin-Derivate der Formel II, worin $R^1$ für H-Phe steht, herstellt.

3. Verfahren nach Anspruch 1 oder 2, dadurch gekennzeichnet, daß man Insulin-Derivate der Formel II herstellt, worin $R^2$ für Gly, Ala, Ser, Thr, Asp oder Glu, insbesondere nur für Asp, steht.

4. Verfahren nach einem oder mehreren der Ansprüche 1 bis 3, dadurch gekennzeichnet, daß man Insulin-Derivate der Formel II herstellt, worin $R^{30}$ für Ala, Thr oder Ser steht.

5. Verfahren nach einem oder mehreren der Ansprüche 1 bis 4, dadurch gekennzeichnet, daß man Insulin-Derivate der Formel II herstellt, worin $R^{31}$ für Arg-OH oder Arg-Arg-OH steht.

6. Verfahren nach einem oder mehreren der Ansprüche 1 bis 5, dadurch gekennzeichnet, daß man Insulin-Derivate der Formel II herstellt, worin X Asn oder Gln bedeutet.

7. Verfahren nach einem oder mehreren der Ansprüche 1 bis 6, dadurch gekennzeichnet, daß man Insulin-Derivate der Formel II herstellt, worin die Sequenzen (A1 bis A20) und (B1 bis B9, B11 bis B29) die Sequenzen des Human-, Schweine- oder Rinderinsulins, insbesondere die Sequenzen des Humaninsulins sind.

8. Verfahren zur Herstellung von Insulin-Derivaten der Formel II gemäß der Definition in Anspruch 1, wobei jedoch

   $R^2$ nur = Asp und
   X = His ist,

   und der physiologisch verträglichen Salze dieser Insulin-Derivate, dadurch gekennzeichnet, daß man Insulin-Derivate der Formel I

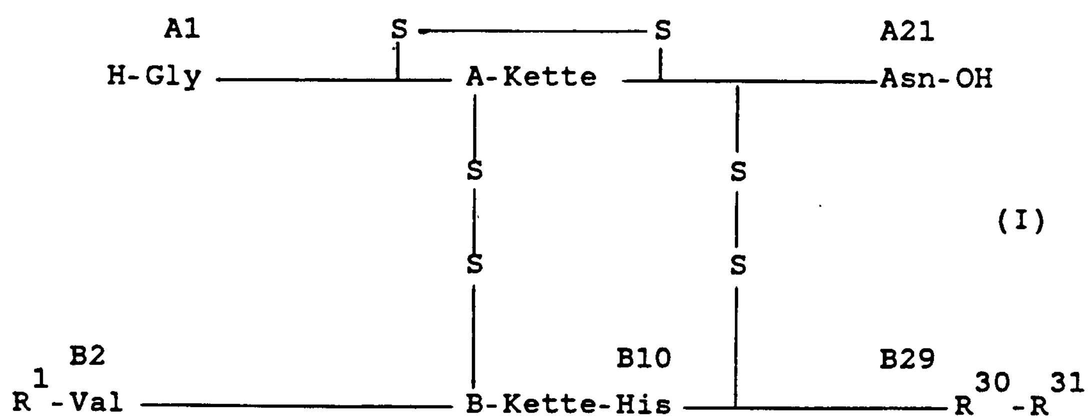

(I)

   worin $R^1$, $R^{30}$ und $R^{31}$ die gleiche Bedeutung wie in Formel II (gemäß der Definition in Anspruch 1) besitzen, in wäßrig-saurem Medium der Hydrolyse unterwirft und die dabei enststehenden Insulin-Derivate der Formel II gegebenenfalls in deren physiologisch verträgliche Salze überführt.

9. Verwendung der Insulin-Derivate der Formel II gemäß der Definition in Anspruch 1 und der physiologisch verträglichen Salze dieser Insulin-Derivate als Wirkstoffe für pharmazeutische Zubereitungen zur Behandlung des Diabetes mellitus.

10. Verfahren zur Herstellung einer pharmazeutischen Zubereitung, dadurch gekennzeichnet, daß man eine wirksame Menge an mindestens einem Insulin-Derivat der Formel II und/oder mindestens einem von deren physiologisch verträglichen Salzen mit einem physiologisch annehmbaren Träger sowie gegebenenfalls mit weiteren Zusatz- und/oder Hilfsstoffen in eine geeignete pharmazeutische Darreichungsform bringt.

11. Verfahren nach Anspruch 10, dadurch gekennzeichnet, daß man der pharmazeutischen Zubereitung noch mindestens eine Zinkverbindung entsprechend einem Gehalt von 1 µg bis 2 mg, vorzugsweise 5

µg bis 200 µg Zink/ml, zusetzt.

**12.** Verfahren nach Anspruch 10 oder 11, dadurch gekennzeichnet, daß man der pharmazeutischen Zubereitung noch ein unmodifiziertes Insulin, vorzugsweise unmodifiziertes Humaninsulin, zusetzt.

**Claims**

**Claims for the following Contracting States : AT, BE, CH, DE, FR, GB, IT, LI, LU, NL, SE**

**1.** An insulin derivative of the formula II

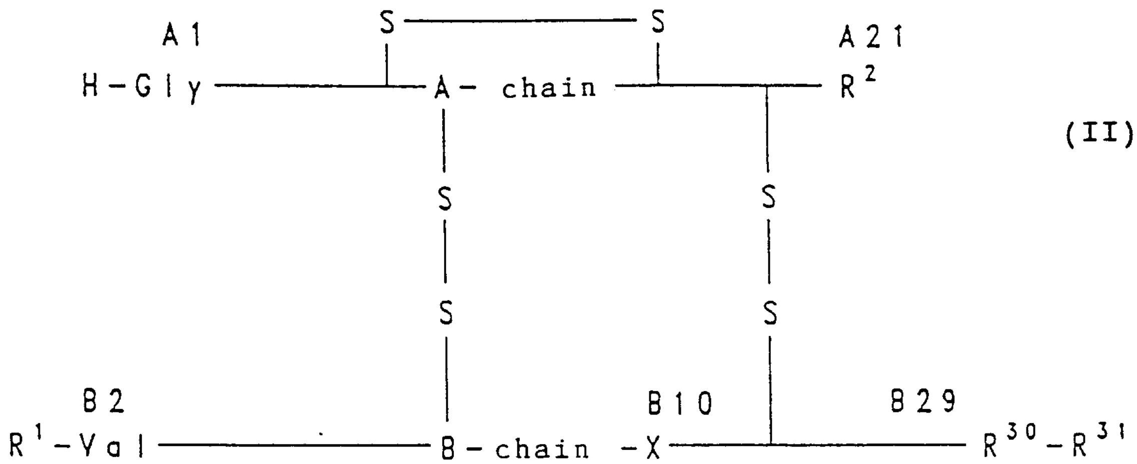

(II)

in which

- $R^1$ denotes H or H-Phe
- $R^2$ denotes a genetically encodable L-amino acid which contains no amide group,
- $R^{30}$ represents the residue of a neutral genetically encodable L-amino acid,
- $R^{31}$ represents a physiologically acceptable organic radical from the group: amino-($C_2$-$C_6$)-alkoxy, ($C_1$-$C_4$)-alkylamino-($C_2$-$C_6$)-alkoxy, di-($C_1$-$C_4$)-alkylamino-($C_2$-$C_6$)-alkoxy, tri-($C_1$-$C_4$)-alkylammonio-($C_2$-$C_6$)-alkoxy, amino-($C_2$-$C_6$)-alkylamino, [($C_1$-$C_4$)-alkylamino]-($C_2$-$C_6$)-alkylamino, di-($C_1$-$C_4$)-alkylamino-($C_2$-$C_6$)-alkylamino or [tri-($C_1$-$C_4$)-alkylamino]-($C_2$-$C_6$)-alkylamino, in particular $-O-[CH_2]_p-NR_2$, $-O-[CH_2]_p-N^{\oplus}R_3$, $-NH[CH_2]_p-NR_2$ or $-NH-[CH_2]_p-N^{\oplus}R_3$, in which $p = 2$ to 6 and R is identical or different and is hydrogen or ($C_1$-$C_4$)-alkyl, or represents 1 to 3 $\alpha$-amino acids, whose terminal carboxyl functionality which is present where appropriate can be free, in the form of an ester functionality, an amide functionality, a lactone or reduced to $CH_2OH$, and
- X represents a genetically encodable L-amino acid,

which has an isoelectric point between 5 and 8.5, and the physiologically tolerated salts thereof.

**2.** An insulin derivative and the physiologically tolerated salts thereof as claimed in claim 1, wherein $R^1$ in formula II represents H-Phe.

**3.** An insulin derivative and the physiologically tolerated salts thereof as claimed in claim 1 or 2, wherein $R^2$ in formula II represents Gly, Ala, Ser, Thr, Asp or Glu, in particular only Asp.

**4.** An insulin derivative and the physiologically tolerated salts thereof as claimed in one or more of claims 1 to 3, wherein $R^{30}$ in formula II represents Ala, Thr or Ser.

**5.** An insulin derivative and the physiologically tolerated salts thereof as claimed in one or more of claims 1 to 4, wherein $R^{31}$ in formula II represents Arg-OH or Arg-Arg-OH.

**6.** An insulin derivative and the physiologically tolerated salts thereof as claimed in one or more of claims 1 to 5, wherein X in formula II denotes Asn or Gln.

**7.** An insulin derivative and the physiologically tolerated salts thereof as claimed in one or more of claims 1 to 6, wherein the sequence (A1 to A20) and (B1 to B9, B11 to B29) in formula II are the sequence of human, porcine or bovine insulin, in particular the sequence of human insulin.

**8.** The use of the insulin derivatives and the physiologically tolerated salts thereof as claimed in one or more of claims 1 to 7, as active substance for pharmaceutical compositions for the treatment of diabetes mellitus.

**9.** A pharmaceutical composition which contains an effective amount of at least one insulin derivative of the formula II and/or at least one of the physiologically tolerated salts thereof as claimed in one or more of claims 1 to 7 in dissolved, amorphous and/or crystalline - preferably in dissolved - form.

**10.** A pharmaceutical composition as claimed in claim 9, which additionally contains 1 $\mu$g to 2 mg, preferably 5 $\mu$g to 200 $\mu$g, of zinc/ml.

**11.** A pharmaceutical composition as claimed in claim 9 or 10, which additionally contains unmodified insulin, preferably unmodified human insulin.

**Claims for the following Contracting States : ES, GR**

**1.** A process for the preparation of an insulin derivative of the formula II

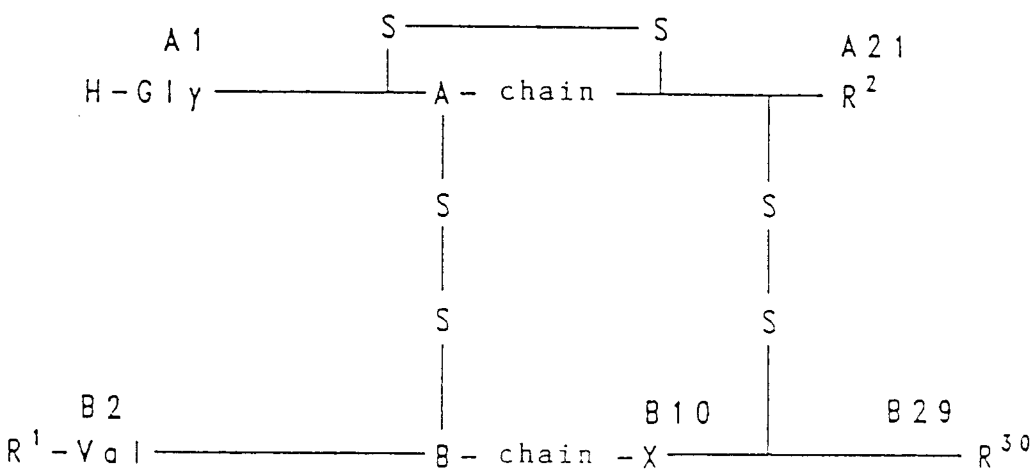

(II)

in which

$R^1$ denotes H or H-Phe,

$R^2$ denotes a genetically encodable L-amino acid which contains no amide group,

$R^{30}$ represents the residue of a neutral genetically encodable L-amino acid,

$R^{31}$ represents a physiologically acceptable organic radical from the group: amino-($C_2$-$C_6$)-alkoxy, ($C_1$-$C_4$)-alkylamino-($C_2$-$C_6$)-alkoxy, di-($C_1$-$C_4$)-alkylamino-($C_2$-$C_6$)-alkoxy, tri-($C_1$-$C_4$)-alkylammonio-($C_2$-$C_6$)-alkoxy, amino-($C_2$-$C_6$)-alkylamino, [($C_1$-$C_4$)-alkylamino]-($C_2$-$C_6$)-alkylamino, di-($C_1$-$C_4$)-alkylamino-($C_2$-$C_6$)-alkylamino or [tri-($C_1$-$C_4$)-alkylamino]-($C_2$-$C_6$)-alkylamino, in particular $-O-[CH_2]_p-NR_2$, $-O-[CH_2]_p-N^{\oplus}R_3$, $-NH[CH_2]_p-NR_2$ or $-NH-[CH_2]_p-N^{\oplus}R_3$, in which p = 2 to 6 and R is identical or different and is hydrogen or ($C_1$-$C_4$)-alkyl, or represents 1 to 3 $\alpha$-amino acids, whose terminal carboxyl functionality which is present where appropriate can be free, in the form of an ester functionality, an amide functionality, a lactone or reduced to $CH_2OH$, and

X represents a genetically encodable L-amino acid,

which has an isoelectric point between 5 and 8.5, and the physiologically tolerated salts thereof wherein the expression of a gene structure coding for this insulin derivative is brought about in a host cell, preferably in a bacterium or in a yeast, and - if the gene structure codes for a fusion protein - the respective insulin derivative is liberated from the fusion protein obtained and converted if required into a physiologically tolerated salt.

**2.** The process as claimed in claim 1, which comprises preparing an insulin derivative of the formula II in which $R^1$ represents H-Phe.

**3.** The process as claimed in claim 1 or 2, which comprises preparing an insulin derivative of the formula II in which $R^2$ represents Gly, Ala, Ser, Thr, Asp or Glu, in particular only Asp.

**4.** The process as claimed in one or more of claims 1 to 3, which comprises preparing an insulin derivative of the formula II in which $R^{30}$ represents Ala, Thr or Ser.

**5.** The process as claimed in one or more of claims 1 to 4, which comprises preparing an insulin derivative of the formula II in which $R^{31}$ represents Arg-OH or Arg-Arg-OH.

**6.** The process as claimed in one or more of claims 1 to 5, which comprises preparing an insulin derivative of the formula II in which X denotes Asn or Gln.

**7.** The process as claimed in one or more of claims 1 to 6, which comprises preparing an insulin derivative of the formula II in which the sequence (A1 to A20) and (B1 to B9, B11 to B29) are the sequence of human, porcine or bovine insulin, in particular the sequence of human insulin.

**8.** A process for the preparation of an insulin derivative of the formula II as defined in claim 1, but where

$R^2$ is only Asp and
X is His,

and of the physiologically tolerated salts of this insulin derivative, which comprises subjecting an insulin derivative of the formula I

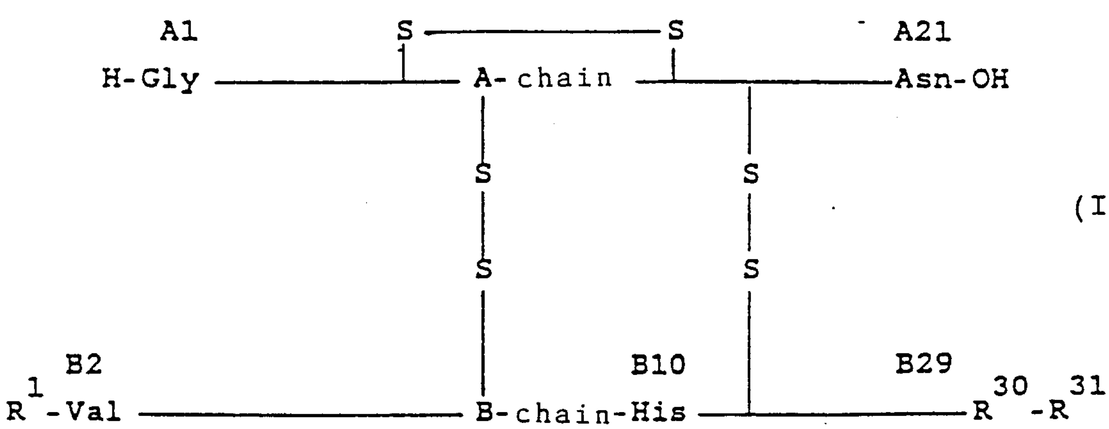

(I)

in which $R^1$, $R^{30}$ and $R^{31}$ have the same meaning as in formula II (as defined in claim 1), to hydrolysis in aqueous acidic medium, and converting the insulin derivative of the formula II produced thereby if required into the physiologically tolerated salts thereof.

**9.** The use of insulin derivatives of the formula II as defined in claim 1 and of the physiologically tolerated salts of these insulin derivatives as active substances for pharmaceutical compositions for the treatment of diabetes mellitus.

**10.** A process for the preparation of a pharmaceutical composition, which comprises converting an effective amount of at least one insulin derivative of the formula II and/or at least one of its physiologically tolerated salts with a physiologically acceptable vehicle and, where appropriate, with further additives and/or auxiliaries into a suitable pharmaceutical presentation.

**11.** The process as claimed in claim 10, wherein at least one zinc compound is also added, corresponding to a content of 1 µg to 2 mg, preferably 5 µg to 200 µg, of zinc/ml to the pharmaceutical composition.

**12.** The process as claimed in claim 10 or 11, wherein an unmodified insulin, preferably unmodified human insulin, is also added to the pharmaceutical composition.

**Revendications**

**Revendications pour les Etats contractants suivants : AT, BE, CH, DE, FR, GB, IT, LI, LU, NL, SE**

**1.** Dérivés d'insuline de formule II

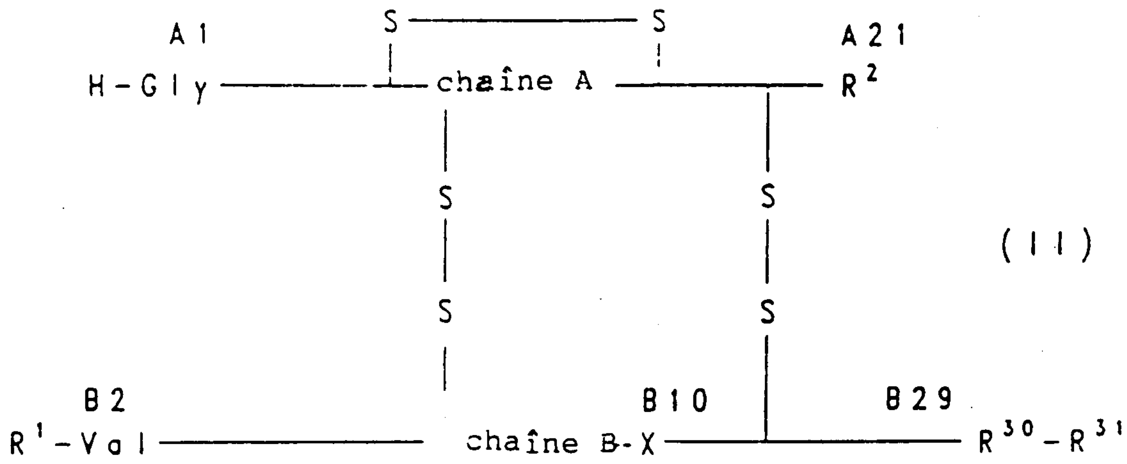

(II)

dans laquelle

$R^1$ représente H ou H-Phe,

$R^2$ représente un L-aminoacide génétiquement codable, ne contenant pas de groupe amido,

$R^{30}$ représente le reste d'un L-aminoacide neutre, génétiquement codable,

$R^{31}$ représente un groupe organique physiologiquement acceptable choisi parmi les groupes amino-alcoxy($C_2$-$C_6$), alkyl($C_1$-$C_4$)amino-alcoxy($C_2$-$C_6$), dialkyl($C_1$-$C_4$)aminoalcoxy($C_2$-$C_6$), tri-alkyl($C_1$-$C_4$)ammonio-alcoxy($C_2$-$C_6$), amino-alkyl($C_2$-$C_6$)amino, [alkyl($C_1$-$C_4$)amino]alkyl($C_2$-$C_6$)amino, dialkyl($C_1$-$C_4$)amino-alkyl($C_2$-$C_6$)amino ou [trialkyl($C_1$-$C_4$)amino]alkyl($C_2$-$C_6$)amino, en particulier $-O-(CH_2)_p-NR_2$, $-O-(CH_2)_p-N^{\oplus}R_3$, $-NH-(CH_2)_p-NR_2$ ou $-NH-(CH_2)_p-N^{\oplus}R_3$, p allant de 2 à 6 et les radicaux R étant identiques ou différents et représentant un atome d'hydrogène ou un groupe alkyle en $C_1$-$C_4$, ou
de 1 à 3 $\alpha$-aminoacides dont la fonction carboxyterminale éventuellement présente peut se trouver sous forme libre, sous forme de fonction ester, sous forme de fonction amide, sous forme de lactone ou réduite en $CH_2OH$, et

X représente un L-aminoacide génétiquement codable,
caractérisés par un point isoélectrique compris entre 5 et 8,5, et sels physiologiquement acceptables de ceux-ci.

**2.** Dérivés d'insuline et leurs sels physiologiquement acceptables selon la revendication 1, caractérisés en ce que, dans la formule II, $R^1$ représente H-Phe.

**3.** Dérivés d'insuline et leurs sels physiologiquement acceptables selon la revendication 1 ou 2, caractérisés en ce que, dans la formule II, $R^2$ représente Gly, Ala, Ser, Thr, Asp ou Glu, en particulier seulement Asp.

**4.** Dérivés d'insuline et leurs sels physiologiquement acceptables selon une ou plusieurs des revendications 1 à 3, caractérisés en ce que, dans la formule II, $R^{30}$ représente Ala, Thr ou Ser.

**5.** Dérivés d'insuline et leurs sels physiologiquement acceptables selon une ou plusieurs des revendications 1 à 4, caractérisés en ce que, dans la formule II, $R^{31}$ représente Arg-OH ou Arg-Arg-OH.

**6.** Dérivés d'insuline et leurs sels physiologiquement acceptables selon une ou plusieurs des revendications 1 à 5, caractérisés en ce que, dans la formule II, X représente Asn ou Gln.

**7.** Dérivés d'insuline et leurs sels physiologiquement acceptables selon une ou plusieurs des revendications 1 à 6, caractérisés en ce que, dans la formule II, les séquences (A1 à A20) et (B1 à B9, B11 à B29) sont les séquences de l'insuline humaine, porcine ou bovine, en particulier les séquences de

l'insuline humaine.

**8.** Utilisation des dérivés d'insuline et de leurs sels physiologiquement acceptables selon une ou plusieurs des revendications 1 à 7, en tant que substances actives pour des compositions pharmaceutiques pour le traitement du diabète sucré.

**9.** Composition pharmaceutique, caractérisée par une quantité efficace d'au moins un dérivé d'insuline de formule II et/ou d'au moins un de ses sels physiologiquement acceptables selon une ou plusieurs des revendications 1 à 7, sous forme dissoute, amorphe et/ou cristalline - de préférence sous forme dissoute.

**10.** Composition pharmaceutique selon la revendication 9, caractérisée par une teneur supplémentaire en zinc allant de 1 µg à 2 mg, de préférence de 5 µg à 200 µg/ml.

**11.** Composition pharmaceutique selon la revendication 9 ou 10, caractérisée par une teneur supplémentaire en insuline non modifiée, de préférence en insuline humaine non modifiée.

**Revendications pour les Etats contractants suivants : ES, GR**

**1.** Procédé pour la préparation de dérivés d'insuline de formule II

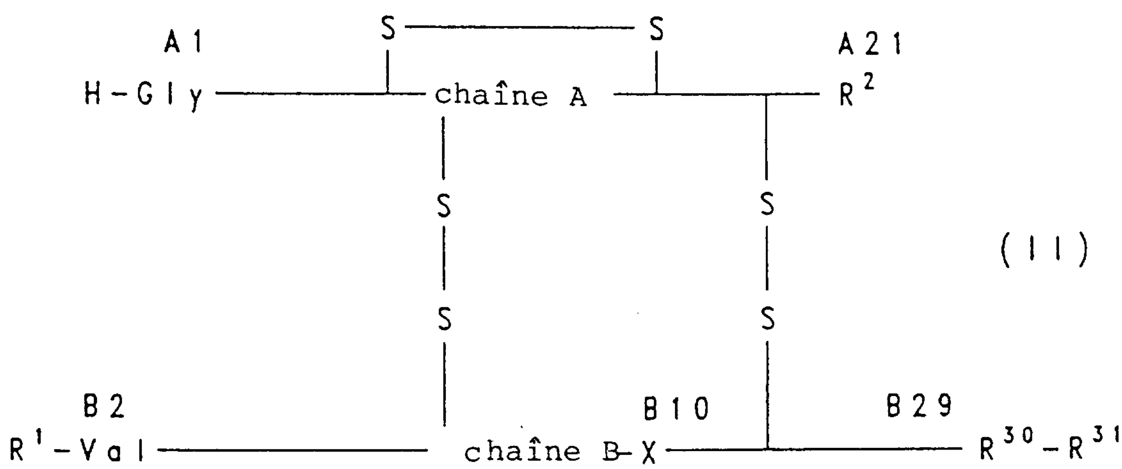

(II)

dans laquelle

- $R^1$ représente H ou H-Phe,
- $R^2$ représente un L-aminoacide génétiquement codable, ne contenant pas de groupe amido,
- $R^{30}$ représente le reste d'un L-aminoacide neutre, génétiquement codable,
- $R^{31}$ représente un groupe organique physiologiquement acceptable choisi parmi les groupes amino-alcoxy($C_2$-$C_6$), alkyl($C_1$-$C_4$)amino-alcoxy($C_2$-$C_6$), dialkyl($C_1$-$C_4$)aminoalcoxy($C_2$-$C_6$), trialkyl($C_1$-$C_4$)-ammonio-alcoxy($C_2$-$C_6$), amino-alkyl($C_2$-$C_6$)amino, [alkyl($C_1$-$C_4$)amino]alkyl($C_2$-$C_6$)amino, dialkyl($C_1$-$C_4$)amino-alkyl($C_2$-$C_6$)amino ou [trialkyl($C_1$-$C_4$)amino]alkyl($C_2$-$C_6$)amino, en particulier $-O-(CH_2)_p-NR_2$, $-O-(CH_2)_p-N^{\oplus}R_3$, $-NH-(CH_2)_p-NR_2$ ou $-NH-(CH_2)_p-N^{\oplus}R_3$, p allant de 2 à 6 et les radicaux R étant identiques ou différents et représentant un atome d'hydrogène ou un groupe alkyle en $C_1$-$C_4$, ou
  de 1 à 3 α-aminoacides dont la fonction carboxyterminale éventuellement présente peut se trouver sous forme libre, sous forme de fonction ester, sous forme de fonction amide, sous forme de lactone ou réduite en $CH_2OH$, et X représente un L-aminoacide génétiquement codable,

ayant un point isoélectrique compris entre 5 et 8,5, et des sels physiologiquement acceptables de ces dérivés d'insuline,

caractérisé en ce que l'on exprime dans une cellule hôte, de préférence dans une bactérie ou dans une levure, des structures géniques codant pour ces dérivés d'insuline et - lorsque les structures géniques codent pour une protéine de fusion - on libère le dérivé d'insuline de formule II respectif à partir de la protéine de fusion obtenue, et éventuellement on le convertit en un sel physiologiquement acceptable.

2. Procédé selon la revendication 1, caractérisé en ce que l'on prépare des dérivés d'insuline de formule II dans lesquels $R^1$ représente H-Phe.

3. Procédé selon la revendication 1 ou 2, caractérisé en ce que l'on prépare des dérivés d'insuline de formule II dans lesquels $R^2$ représente Gly, Ala, Ser, Thr, Asp ou Glu, en particulier seulement Asp.

4. Procédé selon une ou plusieurs des revendications 1 à 3, caractérisé en ce que l'on prépare des dérivés d'insuline de formule II dans lesquels $R^{30}$ représente Ala, Thr ou Ser.

5. Procédé selon une ou plusieurs des revendications 1 à 4, caractérisé en ce que l'on prépare des dérivés d'insuline de formule II dans lesquels $R^{31}$ représente Arg-OH ou Arg-Arg-OH.

6. Procédé selon une ou plusieurs des revendications 1 à 5, caractérisé en ce que l'on prépare des dérivés d'insuline de formule II dans lesquels X représente Asn ou Gln.

7. Procédé selon une ou plusieurs des revendications 1 à 6, caractérisés en ce que l'on prépare des dérivés d'insuline de formule II dans lesquels les séquences (A1 à A20) et (B1 à B9, B11 à B29) sont les séquences de l'insuline humaine, porcine ou bovine, en particulier les séquences de l'insuline humaine.

8. Procédé pour la production de dérivés d'insuline de formule II selon la définition dans la revendication 1, mais dans lesquels
   $R^2$ ne représente que Asp et
   X = His,
   et des sels physiologiquement acceptables de ces dérivés d'insuline, caractérisé en ce que l'on soumet à l'hydrolyse en milieu aqueux-acide des dérivés d'insuline de formule I

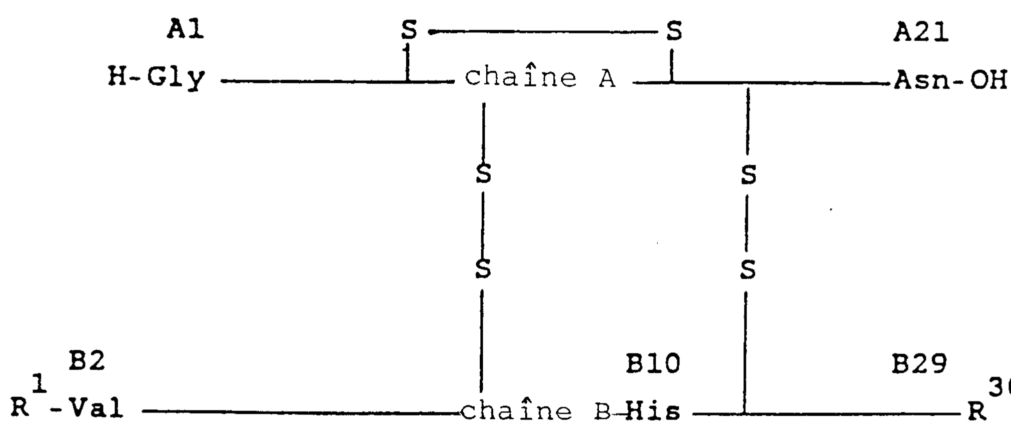

(I)

dans laquelle $R^1$, $R^{30}$ et $R^{31}$ ont les mêmes significations que dans la formule II (selon la définition donnée dans la revendication 1) et on convertit éventuellement en leurs sels physiologiquement acceptables les dérivés d'insuline de formule II ainsi obtenus.

9. Utilisation des dérivés d'insuline de formule II selon la définition dans la revendication 1 et des sels physiologiquement acceptables de ces dérivés d'insuline, en tant que substances actives pour des compositions pharmaceutiques pour le traitement du diabète sucré.

10. Procédé pour la préparation d'une composition pharmaceutique, caractérisé en ce que l'on met sous une forme pharmaceutique appropriée une quantité efficace d'au moins un dérivé d'insuline de formule II et/ou d'au moins l'un de ses sels physiologiquement acceptables, avec un véhicule physiologiquement acceptable, ainsi qu'éventuellement d'autres additifs et/ou adjuvants.

11. Procédé selon la revendication 10, caractérisé en ce que l'on ajoute à la composition pharmaceutique encore au moins un composé à base de zinc correspondant à une teneur en zinc de 1 $\mu$g à 2 mg, de préférence de 5 $\mu$g à 200 $\mu$g/ml.

**12.** Procédé selon la revendication 10 ou 11, caractérisé en ce que l'on ajoute encore à la composition pharmaceutique une insuline non modifiée, de préférence une insuline humaine non modifiée.